(19) Europäisches Patentamt / European Patent Office / Office européen des brevets

(11) **EP 3 709 302 B1**

(12) **EUROPEAN PATENT SPECIFICATION**

(45) Date of publication and mention
of the grant of the patent:
**14.02.2024   Bulletin 2024/07**

(21) Application number: **20162215.6**

(22) Date of filing: **10.03.2020**

(51) International Patent Classification (IPC):
**G16B 25/20** (2019.01)     **C12N 15/09** (2006.01)

(52) Cooperative Patent Classification (CPC):
**G16B 40/30; G16B 45/00; G16C 20/30;
G16C 20/80;** C12N 15/09; G16B 25/20; G16C 20/70

(54) **ESTIMATION METHOD**

SCHÄTZVERFAHREN

PROCÉDÉ D'ESTIMATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.03.2019   JP 2019046887
24.12.2019   JP 2019232236
31.01.2020   JP 2020015263**

(43) Date of publication of application:
**16.09.2020   Bulletin 2020/38**

(73) Proprietor: **Ricoh Company, Ltd.
Ohta-ku
Tokyo 143-8555 (JP)**

(72) Inventors:
• **JI, Yunong
Tokyo 143-8555 (JP)**
• **UNNO, Hirotaka
Tokyo 143-8555 (JP)**
• **HATADA, Shigeo
Tokyo 143-8555 (JP)**
• **SEO, Manabu
Tokyo 143-8555 (JP)**

(74) Representative: **J A Kemp LLP
80 Turnmill Street
London EC1M 5QU (GB)**

(56) References cited:
• **Anonymous: "EURACHEM / CITAC Guide:
Quantifying Uncertainty in Analytical
Measurement Third Edition", , 1 January 2012
(2012-01-01), XP055713759, ISBN:
978-0-948926-30-3 Retrieved from the Internet:
URL:https://www.eurachem.org/images/storie
s/Guides/pdf/QUAM2012_P1.pdf [retrieved on
2020-07-10]**
• **LIANHUA DONG ET AL: "Comparison of four
digital PCR platforms for accurate quantification
of DNA copy number of a certified plasmid DNA
reference material", SCIENTIFIC REPORTS, vol.
5, 25 August 2015 (2015-08-25), page 13174,
XP055340722, DOI: 10.1038/srep13174**
• **CORBISIER PHILIPPE ET AL: "DNA copy number
concentration measured by digital and droplet
digital quantitative PCR using certified reference
materials", CORESTA PTM TECHNICAL
REPORT, SPRINGER BERLIN HEIDELBERG, DE,
vol. 407, no. 7, 20 January 2015 (2015-01-20),
pages 1831-1840, XP035454453, ISSN: 1618-2642,
DOI: 10.1007/S00216-015-8458-Z [retrieved on
2015-01-20]**
• **CRISTIANO IALONGO: "Confidence interval for
quantiles and percentiles", BIOCHEMICA
MEDICA, vol. 29, no. 1, 24 December 2018
(2018-12-24), pages 5-17, XP055713961, HR ISSN:
1330-0962, DOI: 10.11613/BM.2019.010101**
• **Stmu Anonymous: "EURACHEM / CITAC Guide
Setting and Using Target Uncertainty in Chemical
Measurement", , 1 January 2015 (2015-01-01),
XP055714113, Retrieved from the Internet:
URL:https://www.eurachem.org/images/storie
s/Guides/pdf/STMU_2015_EN.pdf [retrieved on
2020-07-13]**

EP 3 709 302 B1

• Bertil Magnusson ET AL: "Nordic Innovation Calculation of Measurement Uncertainty in Environmental Laboratories", , 1 May 2012 (2012-05-01), XP055714242, Retrieved from the Internet: URL:http://www.nordtest.info/images/docume nts/nt-technical-reports/nt_tr_537_ed3_1_E nglish_Handbook%20for%20Calculation%20of %2 0Measurement%20uncertainty%20in%20enviro nm ental%20laboratories.pdf [retrieved on 2020-07-13]

## EP 3 709 302 B1

**Description**

BACKGROUND

Field of the Invention

The present disclosure relates to a technique for quantitative determination of a material stored in a container.

Description of the Related Art

To measure the detection limit of a genetic testing device or create a calibration curve for a low-concentration region for quantitative determination, it is necessary to use a low-copy-number nucleic acid reference material with guaranteed accuracy for quantitative determination. As a method of creating a quantified nucleic acid reference material, a method of performing limiting dilution of a DNA (deoxyribo nucleic acid) solution, or a method of dispensing cells containing DNAs using ink-jet is used, for example.

The limiting dilution method is known to provide a diluted concentration that obeys a Poisson distribution. Such a distribution can be approximated to a normal distribution if the concentration is relatively high. However, if the concentration is low (in particular, if the copy number of nucleic acid molecules is 1 to 100), the right side (i.e., positive side) of the distribution becomes relatively long. Further, there is a relatively prominent tendency that the distribution becomes discrete.

Japanese Patent Application No. 2018-096636 describes a method of dispensing cells using ink-jet. This method does not have a cell measurement accuracy of 100 % during dispensing, and the resulting concentration distribution is asymmetrical, with the right side longer than the left side due to aggregation of cells, for example. In addition, such a distribution is not a common distribution like a Poisson distribution that can be represented by a mathematical expression.

To show the product specifications of a nucleic acid reference material, it is necessary to present the concentration of the nucleic acid reference material included in the product. Specifically, specification values representing the representative concentration (typically, a mean value is used) and variation in the concentration are important. In particular, a smaller specification value regarding variation means higher concentration accuracy. Thus, it is important to use an adequate method of representing variation. In addition, the concentration distribution of a nucleic acid reference material may differ depending on a method of creating the material, and some concentration distribution may not be able to be represented using a common distribution. Thus, it would be desirable to use a method capable of computing a concentration irrespective of the shape of a distribution. That is, it is necessary to compute a concentration using a non-parametric method.

As a method of computing and displaying the product specifications of a reference material, a method that is based on the assumption that variation in the concentration obeys a normal distribution is known (hereinafter referred to as a normal distribution method). The normal distribution method is a method of estimating an interval in which the concentration of a reference material is in the range of the mean value $\pm 2\sigma$ (where $\sigma$ is the standard deviation of the concentration distribution). This method assumes that the concentration distribution obeys a normal distribution and thus is said to be one of the parametric methods.

A percentage-point method, which is used to compute a process capability index, identifies a probability distribution function or a p % point and a (1 - p) % point on a probability distribution with a given shape. The p % point means a point where the probability of occurrence of a phenomenon having a given value or less is p %. For example, the 2.275 % point and the 97.725 % point on a standard normal distribution correspond to - 2 and + 2, respectively. In addition, when a cumulative probability for an interval [p % point, (1 - p) % point] is to satisfy a target probability $\alpha$, p = (1 - $\alpha$) $\times$ 100/2. Percentage points can be used irrespective of the shape of a probability distribution. Thus, percentage points can be used to determine a concentration in a non-parametric way. When the way of thinking a process capability index based on the percentage-point method is used, for example, it is possible to determine an interval in which the probability that the concentration is within the range of a non-normal distribution is 95.45 %, which is equivalent to the mean value $\pm 2\sigma$ on a normal distribution, for example.

WO 2006/030822 describes the following technique as a technique of correcting unevenness in a data distribution of a DNA chip: "[U]nevenness in data on a DNA chip is adequately detected and, if possible, corrected. In a gene expression data processing method for processing array data obtained on the basis of the expression level of genes on a DNA chip and thus obtaining analyzable data, the DNA chip is divided into small regions. The values of data forming the array data are standardized (step 300). The mean value of the standardized data values or the standard deviation of the median is calculated for each small region (step 310). The presence or absence of unevenness in the data on the DNA chip is detected on the basis of an increase in the standard deviation (step 310)" (see Abstract).

Japanese Unexamined Patent Application Publication No. 2004-257809 describes a technique of, based on an object of "providing a standard plastic material prepared by dispersing a known amount of a known element in a plastic base material, the standard plastic material having a predetermined thickness and being capable of providing an accurate analysis result, and a production method therefor," "irradiating a plurality of portions of the standard plastic material with predetermined exciting beams to suppress variation in the intensity of fluorescent X rays generated from the element to less than or equal to 10 % of the relative standard deviation" (see Abstract).

Citation List

Patent Literature

Patent document 1: Japanese Patent Application No. 2018-096636
Patent document 2: WO 2006/030822
Patent document 3: Japanese Unexamined Patent Application Publication No. 2004-257809

SUMMARY

When a concentration is computed based on the assumption that the resulting concentration distribution obeys a normal distribution, the computation result for a low-concentration reference material sample, in particular, may greatly deviate from the actual concentration. This occurs when the actual concentration distribution is bilaterally asymmetrical and a relatively large part of the distribution is not within the range of the mean value $\pm 2\sigma$, for example. Alternatively, even when the actual concentration distribution concentrates in a range narrower than the mean value $\pm 2\sigma$, the computation result will greatly deviate from the actual concentration. Further, when a normal distribution is assumed, the representative value of the concentration distribution has a decimal point and numbers following the decimal point, but the concentration of a low-concentration reference material sample is represented by an integer value (that is, the copy number of molecules). Thus, it would be unnatural to present the numbers following the decimal point as the values of the product specifications. Such problems are difficult to be solved with the technique described in Japanese Patent Application No. 2018-096636.

The aforementioned percentage-point method is a non-parametric method. Thus, it can also be used for concentration distributions other than normal distributions. Meanwhile, the method determines percentage points that are bilaterally symmetrical irrespective of a distribution (for example, the 2.25 % point and the 97.75 % point). Thus, when the actual concentration distribution is an asymmetrical, discrete distribution, an increase in the percentage on one side of the distribution is sharp (Example in Table 1: the 37.07 % point follows the 0.43 % point), and thus, the computation result may include a redundant interval.

In WO 2006/030822, the representative value and the standard deviation of a probability distribution are used, and the data distribution is evaluated based on the premise that the probability distribution is bilaterally symmetrical. Thus, the method is considered to have problems similar to Japanese Patent Application No. 2018-096636 in terms of the shape of the non-parametric probability distribution. Japanese Unexamined Patent Application Publication No. 2004-257809 is also considered to have similar problems to Japanese Patent Application No. 2018-096636 as it uses the relative standard deviation of a probability distribution.

In view of the foregoing, exemplarily embodiments are described below.

The invention is defined by the appended claims.

BRIEF DESCRIPTION OF THE DRAWINGS

FIG. 1 is a perspective view of a reference material sample 1;
FIG. 2A is a graph illustrating the concentration distribution of a reference material contained in the reference material sample 1;
FIG. 2B is a schematic diagram for comparing the probability distribution of FIG. 2A with a normal distribution; and
FIG. 3 is a flowchart illustrating the procedures for computing the product specifications of the reference material sample 1.

DETAILED DESCRIPTION

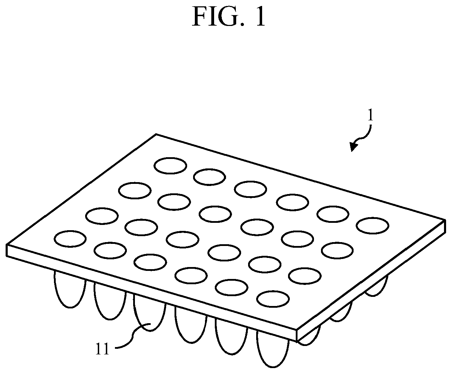

FIG. 1 is a perspective view of a reference material sample 1 according to an embodiment of the present disclosure. The reference material sample 1 includes one or more containers 11 (also referred to as wells) on a plate.

Each container 11 stores a reference material that has been quantified in advance. For example, the container 11 can store a predetermined number of DNA molecules each having a target base sequence for use in an analytical operation. In such a case, storing a predetermined number of copied DNA molecules in each container 11 can create the reference material sample 1. Material samples other than the DNA molecules will be described later.

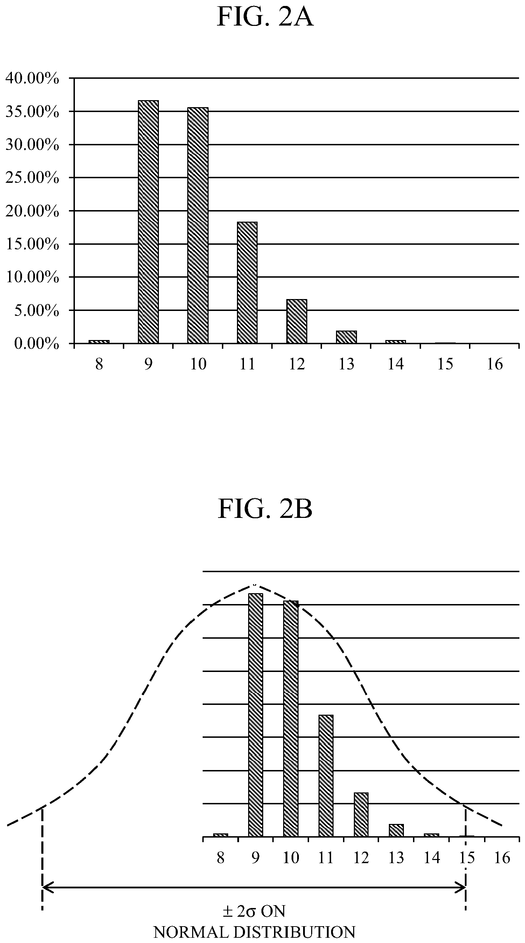

FIG. 2A is a graph illustrating an exemplary concentration distribution of the reference material contained in the reference material sample 1. Herein, the graph illustrates the probability distribution of the copy number of DNA molecules stored in a single container 11. In the example of FIG. 2A, the probability that 9 DNA molecules are stored in a single container 11 is the highest, and the probability that 10 DNA molecules is stored is the second highest. The probabilities of the other numbers of DNA molecules are significantly lower than that of the 9 or 10 DNA molecules. Thus, 10 can be determined as the representative value, for example.

To produce the reference material sample 1, a target probability is set in advance. In the example of FIG. 2A, suppose that the reference material sample 1 was produced so that the probability that 9 to 12 copies of DNA molecules are stored in a single container 11 would become 95.45 %, for example. As the target probability in such a case is 95.45 %, a concentration interval corresponding to the target probability is expressed as [9, 12] (the copy number of molecules, the representative value = 10), for example. Such numerical values can be presented as the product specifications of the reference material sample 1.

A probability distribution such as the one illustrated in FIG. 2A has the following characteristics. As seen in FIG. 2A, the shape of the distribution is not bilaterally symmetrical about the representative value as the center. In particular, a low-concentration sample (for which the concentration is represented by the copy number of molecules) tends to have a longer distribution on its lower-probability portion on the right side (i.e., the side of a greater copy number). Further, since the reference material sample 1 has been created by copying DNA molecules, the abscissa axis of the concentration distribution represents the copy number. That is, the concentration distribution is a discrete distribution. According to such characteristics, the concentration distribution of the low-concentration sample represented by the copy number of molecules appears very different from a normal distribution.

FIG. 2B is a schematic diagram for comparing the probability distribution of FIG. 2A with a normal distribution. When a method based on a normal distribution is used for a sample with a concentration distribution such as the one illustrated in FIG. 2A, the following inconvenience occurs. Suppose a case where an interval in which the concentration is in the range of $\pm 2\sigma$, for example, based on a normal distribution, is presented as the product specifications of the reference material sample 1. However, as illustrated in FIG. 2B, a major part of the actual concentration distribution is not in compliance with the normal distribution on the left side (i.e., the side of a smaller copy number), in particular. Therefore, display of the concentration based on a normal distribution is not considered to be appropriate as the product specifications.

Specifically, the following case would cause inconveniences. In FIG. 2B, if a relatively large part of the actual concentration distribution is located outside of the range of $\pm 2\sigma$ on the normal distribution, the actual concentration distribution that is included in the range of $\pm 2\sigma$ on the normal distribution may become smaller than the original target probability. Alternatively, if the actual concentration distribution concentrates around the representative value and on one side, the target probability may be met within a range narrower than $\pm 2\sigma$ on the normal distribution. In such a case, if the range of $\pm 2\sigma$ on the normal distribution is presented as the product specifications, it follows that a range greater than the target probability is presented as the product specifications. That is, an excessively wide concentration interval is presented as the product specifications. Further, the representative value and the interval of $\pm 2\sigma$ on the normal distribution are not necessarily integers, whereas the representative value and the interval on the actual concentration distribution are integers, thus causing a mismatch between the two.

The present embodiment provides a technique for solving the foregoing inconveniences. That is, the present embodiment aims at adequately computing and displaying the product specifications of the reference material sample 1 according to the actual concentration distribution even when the concentration distribution is bilaterally asymmetrical and discrete. Accordingly, it is considered that the product specifications of, in particular, a low-concentration sample that is represented by the copy number of molecules, for example, can be presented more adequately than is conventionally done.

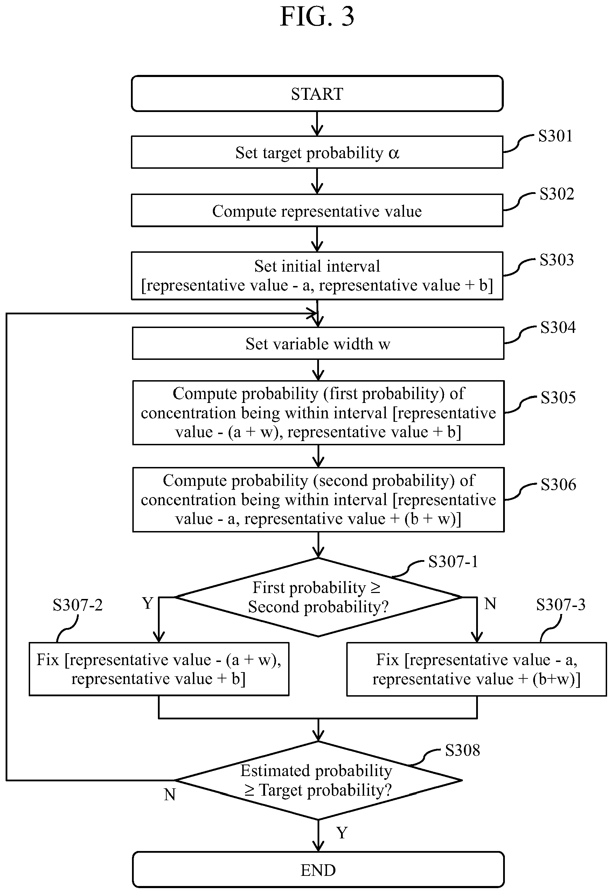

FIG. 3 is a flowchart illustrating the procedures for computing the product specifications of the reference material sample 1 in the present embodiment. The flowchart of FIG. 3 can be carried out either manually or through implementation of each step with hardware, such as a circuit device, or implementation of each step with software and execution of the step with an arithmetic unit. Each step in FIG. 3 will be described below.

(FIG. 3: Step S301)

A target probability for the reference material sample 1 is set. When a target probability equal to that of $\pm 2\sigma$ on a normal distribution is set, for example, such a target probability is 95.45 %. It is also possible to set other appropriate target probabilities.

(FIG. 3: Step S302)

The representative value on the concentration distribution of the reference material sample 1 is computed. Specifically, the actual concentration distribution of the reference material sample 1 is measured or computed in advance to obtain a concentration distribution such as the one illustrated in FIG. 2A. The following description is also based on FIG. 2A. Then, the representative value on the concentration distribution is determined. For the representative value, an appropriate value may be selected according to the shape of the distribution as described below, or an appropriate value may be selected according the intended use of the reference material sample 1. The representative value may also be selected using other appropriate criteria.

(FIG. 3: Step S303)

An interval [the representative value - a, the representative value + b] is set as an initial interval to execute the following steps. The representative value is the one computed in step S302, and is 10 in the example of FIG. 2A. Symbols a and b are integers greater than or equal to zero, and need not be the same numerical value. For example, an initial interval of [9, 10] is set in the example of FIG. 2A. In such a case, a = 1 and b = 0.

(FIG. 3: Step S304)

A variable width w for changing the initial interval is set. For example, when the initial interval is changed in increments of 1 in the abscissa axis direction in the example of FIG. 2A, w is set to 1 (w = 1). When the copy number is large (for example, 100 copies or more), w may be set to 2 or more (w = 2 or more). With a smaller width w, each interval on the concentration distribution will be checked in detail. Meanwhile, with a larger width w, the computation efficiency can be increased. The following steps are performed based on the assumption that w is 1 (w = 1).

(FIG. 3: Step S304: Supplementary notes)

When this step is executed again (second time or later), the previous width w may be changed. For example, the width w may be initially set large to allow for a general search, and then gradually set smaller to allow for a more detailed search.

(FIG. 3: Step S305)

The cumulative probability of the concentrations included in the interval [the representative value - (a + w), the representative value + b] on the actual concentration distribution is computed. If w = 1, the cumulative probability (i.e., first probability) of the concentrations included in the interval [8, 10] is computed. Specifically, the probabilities of the copy number = 8 to the copy number = 10 are added together.

(FIG. 3: Step S306)

The cumulative probability of the concentrations included in the interval [the representative value - a, the representative value + (b + w)] on the actual concentration distribution is computed. If w = 1, the cumulative probability (i.e., second probability) of the concentrations included in the interval [9, 11] is computed. Specifically, the probabilities of the copy number = 9 to the copy number = 11 are added together.

(FIG. 3: Step S307-1)

The first probability computed in step S305 is compared with the second probability computed in step S306. If the first probability is greater than or equal to the second probability, the process proceeds to step S307-2, and if not, the process proceeds to step S307-3.

(FIG. 3: Step S307-2 to Step S307-3)

If the first probability is greater than or equal to the second probability, it means that extending the interval leftward will allow a more portion of the actual concentration distribution to be included. Therefore, in such a case, the interval set in step S305 is fixed (S307-2). If the first probability is less than the second probability, it means that extending the interval rightward will allow a more portion of the actual concentration distribution to be included. Therefore, in such a case, the interval set in step S306 is fixed (S307-3).

(FIG. 3: Step S308)

Steps S304 to S307 (S307-1 to S307-3) are repeated until the cumulative probability computed in step S305 or S306 becomes greater than or equal to the target probability. The flowchart ends at a time point when the cumulative probability becomes greater than or equal to the target probability.

The product specifications computed according to the flowchart of FIG. 3 can be displayed as the product specifications of the reference material sample 1. For example, a label describing the product specifications can be attached to the reference material sample 1 so that the product specifications can be displayed. That is, the reference material sample 1 includes a display portion displaying the product specifications.

<Computation Example 1>

Table 1 illustrates an example of the actual concentration distribution of the reference material sample 1, which correspond to the real values in FIG. 2A. In the following, product specifications computed according to the procedures described with reference to FIG. 3 and product specifications computed with a conventional method are compared using the reference material sample 1 of Table 1.

[Table 1]

| Copy/Well | Probability |
|-----------|-------------|
| 8 | 0.43 % |
| 9 | 36.64 % |
| 10 | 35.57 % |
| 11 | 18.31 % |
| 12 | 6.62 % |
| 13 | 1.88 % |
| 14 | 0.44 % |
| 15 | 0.09 % |
| 16 | 0.02 % |

Table 2 illustrates the results of the product specifications computed with each method. The representative value of the normal distribution method is the mean value. For the percentage-point method B, a Poisson distribution with a mean value of 10 was used as the concentration distribution. Thus, the mean value (= 10) of the Poisson distribution was selected as the representative value. For the present embodiment and the percentage-point method A, the probabilities of an interval of 9 molecules and an interval of 10 molecules are almost the same. Thus, 10 as the median was selected as the representative value. For each of the percentage-point methods A and B, the target probability $\alpha$ is 95.45 %. Thus, an interval was identified through a search for the 2.275 % point and the 97.725 % point.

[Table 2]

| Method | Probability Distribution | Target Probability $\alpha$ | Representative Value | Estimated Interval | Interval Width | Probability within Interval |
|---|---|---|---|---|---|---|
| Present Embodiment | Non-Parametric | 95.45 % Note: This correspo nds to $\pm 2\sigma$ of the normal distribution. | 10 | [9, 12] | 3 | 97.14 % |
| Percentage-Point Method A | Non-Parametric | | 10 | [9, 13] | 4 | 99.02 % |
| Percentage-Point Method B | Poisson Distribution | | 10 | [4, 17] | 13 | 100.00 % |
| Normal Distribution Method | Normal Distribution | | 10.02 | [7.92, 12.12] | 4.20 | 97.57 % |

As a result, each method is found to achieve the target probability $\alpha$. However, the fixed interval of the normal distribution method has a decimal value. Thus, the cumulative probability of an interval of the closest integers [8, 12] within the interval of the computation result is displayed. As illustrated in Table 2, the computation result of the present embodiment has the narrowest interval width of all the other methods. Thus, the present embodiment is deemed to display the properties of the reference material sample 1 most accurately. For the percentage-point method A, the probability of the copy number being less than the lower limit value 9 is 0.43 %, but a percentage point with a lower probability of 2.275 % was searched for. Thus, a redundant interval width of 2.275 % - 0.43 % = 1.845 % was secured. For this reason, it is considered that the interval width of the estimation result is greater than that of the present embodiment. For the percentage-point method B, the shape of the probability distribution greatly differs from that of the Poisson distribution. For this reason, it is considered that the interval width of the estimation result is significantly greater than that of the present embodiment. For the normal distribution method, an interval that is bilaterally symmetrical about the representative value is the estimation result. Thus, the interval on the left side, in particular, is larger than that of the present embodiment.

<Computation Example 2>

Table 3 illustrates another example of the actual concentration distribution of the reference material sample 1. In the following, product specifications computed according to the procedures described with reference to FIG. 3 and product specifications computed with a conventional method are compared using the reference material sample 1 of Table 3.

[Table 3]

| Copy/Well | Probability |
|---|---|
| 4 | 0.29 % |
| 5 | 41.44 % |
| 6 | 35.86 % |
| 7 | 15.98 % |
| 8 | 4.96 % |
| 9 | 1.19 % |
| 10 | 0.23 % |
| 11 | 0.04 % |
| 12 | 0.01% |

Table 4 illustrates the results of the product specifications computed with each method. The representative value of the normal distribution method is the mean value. For the percentage-point method B, a Poisson distribution with a mean value of 6 was used as the concentration distribution. Thus, the mean value (= 6) of the Poisson distribution was selected as the representative value. For each of the present embodiment and the percentage-point method A, the probability of the mode 5 is obviously higher than those of the other intervals. Thus, the mode 5 was selected as the representative value. For each of the percentage-point methods A and B, the target probability $\alpha$ is 95.45 %. Thus, an interval was identified through a search for the 2.275 % point and the 97.725 % point.

[Table 4]

| Method | Probability Distribution | Target Probability $\alpha$ | Representative Value | Estimated Interval | Interval Width | Probability within Interval |
|---|---|---|---|---|---|---|
| Present Embodiment | Non-Parametric | 95.45 % | 5 | [5, 8] | 3 | 98.24 % |
| Percentage-Point Method A | Non-Parametric | Note: This correspon | 5 | [5, 8] | 3 | 98.24 % |
| Percentage-Point Method B | Poisson Distribution | ds to $\pm 2\sigma$ of the normal distribution. | 6 | [2, 11] | 9 | 99.99 % |
| Normal Distribution Method | Normal Distribution | | 5.89 | [3.96, 7.811 | 3.85 | 93.57 % |

As a result, the present embodiment and the percentage-point method A are found to achieve the target probability $\alpha$. For each of the present embodiment and the percentage-point method A, the interval width is 3, which is the smallest of all. For the percentage-point method B, the shape of the probability distribution greatly differs from that of the Poisson distribution. For this reason, it is considered that the interval width of the estimation result is significantly greater than that of the present embodiment. For the normal distribution method, the fixed interval has decimal values. Thus, an interval of the closest integers [4, 7] within the interval of the computation result is the substantial estimation result. For the normal distribution method, an interval that is bilaterally symmetrical about the representative value is the estimation result. Thus, an interval on the left side, in particular, is large and an interval on the right is insufficient, and for this reason, it is considered that the cumulative probability does not satisfy the target probability $\alpha$.

<Computation Example 3>

Table 5 illustrates the results obtained by computing the product specifications of the reference material sample 1, which has been created with the limiting dilution method, using each method. Unlike Tables 1 to 4, the reference material sample 1 has been created with the limiting dilution method. Thus, the concentration distribution obeys a Poisson distribution. In Table 5, examples in which a single container 11 stores an average of 1 to 10000 DNA molecules were used. Since the concentration distribution obeys a Poisson distribution, there is no difference between the percentage-point methods A and B. Thus, such methods are collectively referred to as a "percentage-point method" in Table 5.

[Table 5]

| Target Probability $\alpha$ | Average Copy Number (Representative Value) | Method | Estimated Interval | Interval Width | Probability within Interval |
|---|---|---|---|---|---|
| | 1 | Present Embodiment | [0, 3] | 3 | 98.10 % |
| | | Percentage-Point Method | [0, 3] | 3 | 98.10 % |
| | | Normal Distribution Method | [-1, 3] | 4 | 100 % |

(continued)

| Target Probability $\alpha$ | Average Copy Number (Representative Value) | Method | Estimated Interval | Interval Width | Probability within Interval |
|---|---|---|---|---|---|
| 95.45 % Note:This corresponds to $\pm 2\sigma$ of the normal distribution. | 10 | Present Embodiment | [4, 16] | 12 | 96.26 % |
| | | Percentage-Point Method | [4, 17] | 13 | 97.54 % |
| | | Normal Distribution Method | [3.68, 16.32] | 12.65 | 96.26 % |
| | 100 | Present Embodiment | [81, 120] | 39 | 95.47 % |
| | | Percentage-Point Method | [81, 120] | 39 | 95.47 % |
| | | Normal Distribution Method | [80, 120] | 40 | 95.99 % |
| | 1000 | Present Embodiment | [937, 1063] | 126 | 95.54 % |
| | | Percentage-Point Method | [937, 1064] | 127 | 95.70 % |
| | | Normal Distribution Method | [936.75, 1063.25] | 126.49 | 95.54 % |
| | 10000 | Present Embodiment | [9801, 10200] | 399 | 95.45 % |
| | | Percentage-Point Method | [9801, 10200] | 399 | 95.45 % |
| | | Normal Distribution Method | [9800, 10200] | 400 | 95.50 % |

As a result, each method is found to achieve the target probability $\alpha$. For each number of molecules, the interval width of the present embodiment is the smallest. Thus, it is found that the present embodiment can also be advantageously used when the concentration distribution obeys a typical Poisson distribution. The interval width according to the present embodiment is smaller than those of the normal distribution method and the percentage-point method by one. In particular, regarding a case where the number of molecules is less than or equal to 100, when the present embodiment is compared with one of the two other methods that has a greater interval width, the interval width of the present embodiment is smaller than that of the other method by 2.5 to 25 %. This can confirm that the present embodiment is particularly advantageous for a low-concentration sample containing an average of 100 molecules or less.

<Conclusion of the Present Embodiment>

The reference material sample 1 according to the present embodiment includes a display portion that displays the product specifications computed according to the procedures described with reference to FIG. 3. As the product specifications, (a) the representative value of the concentration distribution and (b) the lower limit value and the upper limit value representing an interval on the probability distribution can be displayed. Further, (c) the target probability $\alpha$ may also be displayed. The target probability $\alpha$ need not be displayed on the display portion if the numerical value can be known without the display portion such as when the value is determined as the industry standard, for example. Displaying the product specifications via the display portion will allow a user to know the correct specifications of the reference material sample 1.

In the present embodiment, after an initial interval is set on the probability distribution, the initial interval is widened to a side with a higher cumulative probability while being gradually widened to the right or left side by the variable width w. Setting the variable width w according to the properties of the probability distribution can, for a given shape of the probability distribution, estimate with high accuracy the minimum interval for which the cumulative probability is greater than or equal to the target probability $\alpha$. Accordingly, product specifications with higher accuracy than those of conventional methods can be presented for a low-concentration sample containing 100 molecules or less, in particular.

<Another Embodiment 1>

The amount y of the reference material is influenced by other amounts (for example, the concentration $x_1$ of the solution and the volume $x_2$ of the solution dispensed). When a single sample is created by mixing a reference material sample with the distribution of Table 1 and a reference material sample with the distribution of Table 3, a representative value of a new sample as well as an interval having a probability greater than or equal to 95.45 % can be easily estimated according to the following procedures. The result is that the representative value is 15 copies/wells and the estimated interval is [10.30, 14.42]. When the estimated interval is represented by integers, it becomes an interval of the minimum integers [10, 15] that can totally include the estimated interval.

Provided that the amount y of the material is influenced by other amounts $x_1, x_2,..., x_n$, $y = f(x_1, x_2,..., x_n)$, the absolute value of the difference between the representative value $R_y$ of the amount y and the lower limit tolerance is $A_y$, the absolute value of the difference between the upper limit tolerance and the representative value $R_y$ is $B_y$, the absolute value of the difference between the representative value $R_{xi}$ of each of the amounts $x_1, x_2,..., x_n$ and the lower limit tolerance is $A_{x1}, A_{x2},..., A_{xn}$, and the absolute value of the difference between the upper limit tolerance and the representative value $R_{xi}$ is $B_{x1}, B_{x2},..., B_{xn}$, the values of $A_y$, $B_y$, and $R_y$ can be estimated with the following formulae.

[Math. 1]

$$A_y = \sqrt{\sum_{i=1}^{n} \left( \frac{\partial y}{\partial x_i} \cdot A_{xi} \right)^2}$$

$$B_y = \sqrt{\sum_{i=1}^{n} \left( \frac{\partial y}{\partial x_i} \cdot B_{xi} \right)^2}$$

$$R_y = f(R_{x1}, R_{x2}, \cdots, R_{xn})$$

<Another Embodiment 2>

The aforementioned embodiment illustrates an example in which the amount of the reference material contained in the reference material sample is estimated and displayed. Instead, the average amount of each sample of the reference material contained in the reference material sample may also be estimated and displayed. In such a case, the measurement result of the average amount of the reference material has measurement uncertainty according to a probability distribution.

For example, when there are 14 samples of a reference material sample having the distribution of Table 1, the representative value of the average amount of each sample as well as an interval having a probability greater than or equal to 95.45 % can be easily estimated according to the following procedures. The result is that the representative value is 10 copies/wells and the estimated interval is [7.59, 13.21]. When the estimated interval is represented by integers, it becomes an interval of the minimum integers [7, 14] that can totally include the estimated interval.

Provided that the absolute value of the difference between the representative value $R_y$ of the amount y of the material and the lower limit tolerance is $A_y$, the absolute value of the difference between the upper limit tolerance and the representative value $R_y$ is $B_y$, the absolute value of the difference between the representative value $R_{ym}$ of the mean value of the amount y of the material and the lower limit tolerance is $A_{ym}$, the absolute value of the difference between the representative value $R_{ym}$ and the upper limit tolerance is $B_{ym}$, and the number of samples of the material is $N_y$, the values of $A_{ym}$, $B_{ym}$, and $R_{ym}$ can be estimated with the following formulae.

[Math. 2]

$$A_{ym} = \frac{A_y}{\sqrt{N_y}}$$

$$B_{ym} = \frac{B_y}{\sqrt{N_y}}$$

$$R_{ym} = R_y$$

<Another Embodiment 3>

When a given amount of a nucleic acid solution sample is dispensed into each of the 14 wells, the average amount y of the nucleic acid dispensed into each well is influenced by the concentration $x_1$ of the solution, the volume $x_2$ of the solution dispensed, and a deviation $x_3$ attributed to a Poisson distribution. Table 6 illustrates the representative value of each element and an interval having a probability greater than or equal to 95.45 %. Each value can be determined according to the following procedures. Since $y = x_1 \times x_2 + x_3$, the representative value of the average amount of each well is 1 copy/well, and the estimated interval is [0.60, 1.63]. When the estimated interval is represented by integers, it becomes an interval of the minimum integers [0, 2] that can totally include the estimated interval.

[Table 6]

|  | Representative Value | Estimated Interval |
|---|---|---|
| Concentration of Reference Material | 0.25 Copy/$\mu$L | [0.174, 0.331] |
| Volume of Dispensed Solution | 4 $\mu$L | [3.94, 4.06] |
| Deviation Attributed to Poisson Distribution | 0 Copy | [-0.267, 0.535] |

Provided that the amount y of the material is influenced by other amounts $x_1$, $x_2$,..., $x_n$ described above, $y = f(x_1, x_2,..., x_n)$, the absolute value of the difference between the representative value $R_{ym}$ of the mean value of the amount y and the lower limit tolerance is $A_{ym}$, the absolute value of the difference between the representative value $R_{ym}$ and the upper limit tolerance is $B_{ym}$, the absolute value of the difference between the representative value $R_{xm1}$, $R_{xm2}$,..., $R_{xmn}$ of the mean value of each of the amounts $x_1$, $x_2$,..., $x_n$ and the lower limit tolerance is $A_{xm1}$, $A_{xm2}$,..., $A_{xmn}$, and the absolute value of the difference between the representative value $R_{xm1}$, $R_{xm2}$,..., $R_{xmn}$ and the upper limit tolerance is $B_{xm1}$, $B_{xm2}$,..., $B_{xmn}$, the values of $A_{ym}$, $B_{ym}$, and $R_{ym}$ can be computed with the following formulae.

[Math. 3]

$$A_{ym} = \sqrt{\sum_{i=1}^{n} \left( \frac{\partial y}{\partial x_i} \cdot A_{xmi} \right)^2}$$

$$B_{ym} = \sqrt{\sum_{i=1}^{n} \left( \frac{\partial y}{\partial x_i} \cdot B_{xmi} \right)^2}$$

$$R_{ym} = f(R_{xm1}, R_{xm2}, \cdots, R_{xmn})$$

<Another Embodiment 4>

When a calibration curve is created with six types of the designated amounts of a reference material, using a real-time PCR (polymerase chain reaction) for quantitative determination of nucleic acids through nucleic acid amplification, the uncertainty of the average quantitative result y is influenced by variation $x_1$ in the quantitative results of the samples for quantitative determination, variation $x_2$ in the designated amounts of the reference material used to create the calibration curve, and variation $x_3$ in the amplification results of the designated amounts of the reference material for creation of the calibration curve. The representative value of y is the mean value of the quantitative results and thus is known. Since the relationship between y and $x_1$ to $x_3$ cannot be represented by a formula, ay/ax; is typically regarded as 1. Provided that the absolute value of the difference between the representative value $R_{xm}$ of the mean value of each element and the lower limit of the estimated interval is $A_{xm}$, and the absolute value of the difference between the representative value $R_{xm}$ and the upper limit of the estimated interval is $B_{xm}$, each relative value with respect to the representative value $R_{xm}$ is illustrated in Table 7. Each value can be determined according to the following procedures. When the representative value of y is 7.03, the estimated interval of y is [5.35, 9.33].

[Table 7]

|  | $A_{xm}/R_{xm}$ | $B_{xm}/R_{xm}$ |
|---|---|---|
| $x_1$ | 0.220 | 0.287 |
| $x_2$ | 0.034 | 0.125 |
| $x_3$ | 0.085 | 0.094 |

Provided that the amount y of the material is influenced by other amounts $x_1$, $x_2$,..., $x_n$ described above, y = $f(x_1, x_2,..., x_n)$, the absolute value of the difference between the representative value $R_{ym}$ of the mean value of the amount y and the lower limit tolerance is $A_{ym}$, the absolute value of the difference between the representative value $R_{ym}$ and the upper limit tolerance is $B_{ym}$, the absolute value of the difference between the representative value $R_{xm1}$, $R_{xm2}$,..., $R_{xmn}$ of the mean value of each of the amounts $x_1$, $x_2$,..., $x_n$ and the lower limit tolerance is $A_{xm1}$, $A_{xm2}$,..., $A_{xmn}$, and the absolute value of the difference between the representative value $R_{xm1}$, $R_{xm2}$,..., $R_{xmn}$ and the upper limit tolerance is $B_{xm1}$, $B_{xm2}$,..., $B_{xmn}$, the values of $A_{ym}$, $B_{ym}$, and $R_{ym}$ can be estimated with the following formulae.

[Math. 4]

$$A_{ym} = R_{ym} \sqrt{\sum_{i=1}^{n} \left( \frac{\partial y}{\partial x_i} \cdot \frac{A_{xmi}}{R_{xmi}} \right)^2}$$

$$B_{ym} = R_{ym} \sqrt{\sum_{i=1}^{n} \left( \frac{\partial y}{\partial x_i} \cdot \frac{B_{xmi}}{R_{xmi}} \right)^2}$$

$$R_{ym} = f(R_{xm1}, R_{xm2}, \cdots, R_{xmn})$$

To create a calibration curve, six types of a reference material that include 1, 5, 10, 20, 40, and 80 copies/wells are used. Provided that the representative value of the mean value of each reference material is $R_Z$, the absolute value of the difference between the representative value and the lower limit of the estimated interval is $A_Z$, the absolute value of the difference between the representative value and the upper limit of the estimated interval is $B_Z$, and the number of the samples of the reference material is $N_Z$, each relative value with respect to the representative value $R_Z$ is illustrated in Table 8. Each value can be determined according to the following procedures. $A_{xm}/R_{xm}$ and $B_{xm}/R_{xm}$ with respect to the variation $x_2$ in the designated amounts of the reference material used to create the calibration curve are 0.034 and 0.125, respectively.

[Table 8]

|  | $R_Z$ | $A_Z/R_Z$ | $B_Z/R_Z$ | $N_Z$ |
|---|---|---|---|---|
| $Z_1$ | 1 | 0.000 | 0.277 | 14 |
| $Z_2$ | 5 | 0.053 | 0.107 | 14 |
| $Z_3$ | 10 | 0.053 | 0.053 | 14 |
| $Z_4$ | 20 | 0.027 | 0.040 | 14 |
| $Z_5$ | 40 | 0.020 | 0.033 | 14 |
| $Z_6$ | 80 | 0.017 | 0.020 | 14 |

Table 9 illustrates $A_Z/R_Z$, $B_Z/R_Z$, and $N_Z$ obtained by performing an inverse operation on the copy number of nucleic acids of each reference material using the created calibration curve. As a result of the computation performed through the same procedures, it is found that $A_{xm}/R_{xm}$ and $B_{xm}/R_{xm}$ with respect to the variation $x_3$ in the amplification results of the designated amounts of the reference material for creation of the calibration curve are 0.085 and 0.094, respectively.

[Table 9]

|  | $R_Z$ | $A_Z/R_Z$ | $B_Z/R_Z$ | $N_Z$ |
|---|---|---|---|---|
| $Z_1$ | 1.06 | 0.095 | 0.105 | 14 |
| $Z_2$ | 5.00 | 0.109 | 0.123 | 14 |
| $Z_3$ | 9.36 | 0.089 | 0.098 | 14 |
| $Z_4$ | 21.17 | 0.092 | 0.102 | 14 |
| $Z_5$ | 40.64 | 0.057 | 0.060 | 14 |
| $Z_6$ | 81.65 | 0.056 | 0.059 | 14 |

Regarding the plurality of types of material samples having different mean values of the amounts x, provided that the absolute value of the difference between the representative value $R_{Z1}$, $R_{Z2}$,..., $R_{Zn}$ of each of the mean values and the lower limit tolerance is $A_{Z1}$, $A_{Z2}$,..., $A_{Zn}$, the absolute value of the difference between the representative value $R_{Z1}$, $R_{Z2}$,..., $R_{Zn}$ and the upper limit tolerance is $B_{Z1}$, $B_{Z2}$,..., $B_{Zn}$, and the number of the material samples is $N_{zi}$, $N_{Z2}$,..., $N_{Zn}$, the values of $A_{xm}/R_{xm}$ and $B_{xm}/R_{xm}$ can be estimated with the following formulae.

[Math. 5]

$$\frac{A_{xm}}{R_{xm}} = \sqrt{\frac{\sum_{i=1}^{n}(N_{Zi} - 1) \cdot \left(\frac{A_{Zi}}{R_{Zi}}\right)^2}{\sum_{i=1}^{n}(N_{Zi} - 1)}}$$

$$\frac{B_{xm}}{R_{xm}} = \sqrt{\frac{\sum_{i=1}^{n}(N_{Zi} - 1) \cdot \left(\frac{B_{Zi}}{R_{Zi}}\right)^2}{\sum_{i=1}^{n}(N_{Zi} - 1)}}$$

<Regarding modified example of the present embodiment>

The present embodiment has been described above using a low-concentration sample as an example of a reference material with a discrete, bilaterally asymmetrical concentration distribution. For example, the reference material sample 1 created by copying DNA molecules corresponds to such a sample. The present embodiment can also be advantageously used for a concentration distribution that is not discrete and a concentration distribution that is bilaterally symmetrical as described with reference to Computation Example 3.

Although the aforementioned embodiment illustrates a nucleic acid reference material sample as an exemplary material sample having containers 11 each storing a quantified material, the present embodiment is also applicable to a material sample having containers storing other quantified materials. That is, although the aforementioned embodiment illustrates an example in which copied nucleic acid molecules are stored in each container 11, the material stored in each container 11 is not necessarily limited to copied nucleic acid molecules. The present embodiment is also applicable to other material samples because it is applicable to any probability distributions. Further, the present embodiment is also applicable to probability distributions other than concentration probability distributions.

In the foregoing embodiments, the target base sequences of molecules stored in each container 11 may be either the same or different. Further, the target base sequence of each molecule stored in a single container 11 may be either the same or different. For material samples other than DNA molecules, the material composition may also be either the same or different among the containers 11.

Although the aforementioned embodiment exemplarily illustrates a label to be attached to the reference material sample 1 as an example of a display portion, the display portion may be configured in different ways. For example, a piece of paper, such as a manual of a product describing its specifications, may be displayed by being packed together with the reference material sample 1. Further, the product specifications may be displayed by being presented over a network. The product specifications may also be displayed using any other appropriate methods.

In the aforementioned embodiment, information displayed on the display portion may be expressed with any method as long as it can identify product specifications. For example, the lower limit and the upper limit of an interval may be displayed like an "interval [8, 12]," or information that can identify an interval according to some procedures may be presented. This is also true of the target probability $\alpha$ and the representative value.

The reference material sample 1 can be created by ejecting molecules of a material (for example, DNA molecules) into the containers 11 from an ink-jet apparatus (i.e., liquid droplet ejection apparatus), for example (see Japanese Patent Application No. 2018-096636). In such a case, the representative value can be computed using the properties of the ink-jet apparatus in step S302. For example, using properties, such as a single liquid droplet ejection amount and the concentration of a material contained in the liquid droplet, is considered.

Reference Signs List

1: reference material sample
11: container

**Claims**

1. A computer-implemented estimation method for estimating an amount of a quantified material injected into a container by a liquid droplet ejection apparatus, the amount of the material having variation according to a probability distribution, and a probability that the amount of the material is within a range of a lower limit tolerance to an upper limit tolerance on the probability distribution being greater than or equal to a target probability, the estimating method comprising:

calculating the probability distribution, wherein the calculating the probability distribution includes computing the probability distribution using a performance property of the liquid droplet ejection apparatus configured to inject the material into the container,
a first step of setting the target probability;
a second step of computing the representative value of the amount of material on the probability distribution;
a third step of setting an interval of a lower limit to an upper limit including the representative value on the probability distribution;
a fourth step of setting a variable width of the interval;
a fifth step of increasing a size of the interval by the variable width from the representative value toward the lower limit, and computing a first probability that the amount of the material is within the interval with the increased size;
a sixth step of increasing the size of the interval by the variable width from the representative value toward the upper limit, and computing a second probability that the amount of the material is within the interval with the increased size; and

a seventh step of fixing the size of the interval set in the fifth step if the first probability is greater than or equal to the second probability, or fixing the size of the interval set in the sixth step if the first probability is less than the second probability,

wherein:

the fourth step, the fifth step, the sixth step, and the seventh step are repeated until the probability that the amount of the material is within the interval becomes greater than or equal to the target probability,

the lower limit tolerance is the lower limit of the interval at a time point when the probability that the amount of the material is within the interval becomes greater than or equal to the target probability, and

the upper limit tolerance is the upper limit of the interval at a time point when the probability that the amount of the material is within the interval becomes greater than or equal to the target probability; and

wherein the material is nucleic acid molecules.

2. The estimation method of claim 1, wherein provided that

an amount y of the material is influenced by other amounts $x_1$, $x_2$,..., $x_n$,

$$y = f(x_1, x_2, \ldots, x_n),$$

an absolute value of a difference between a representative value $R_y$ of the amount y and the lower limit tolerance is $A_y$,

an absolute value of a difference between the upper limit tolerance and the representative value $R_y$ is $B_y$,

an absolute value of a difference between a representative value $R_{xi}$ of each of the amounts $x_1$, $x_2$,..., $x_n$ and the lower limit tolerance is $A_{x1}$, $A_{x2}$,..., $A_{xn}$, and

an absolute value of a difference between the upper limit tolerance and the representative value $R_{xi}$ is $B_{x1}$, $B_{x2}$,..., $B_{xn}$,

values of $A_y$, $B_y$, and $R_y$ are estimated with the following formulae.

[Math. 1]

$$A_y = \sqrt{\sum_{i=1}^{n} \left(\frac{\partial y}{\partial x_i} \cdot A_{xi}\right)^2}$$

$$B_y = \sqrt{\sum_{i=1}^{n} \left(\frac{\partial y}{\partial x_i} \cdot B_{xi}\right)^2}$$

$$R_y = f(R_{x1}, R_{x2}, \cdots, R_{xn})$$

3. The estimation method according to claim 1, wherein provided that

an absolute value of a difference between a representative value $R_y$ of an amount y of the material and the lower limit tolerance is $A_y$,

an absolute value of a difference between the upper limit tolerance and the representative value $R_y$ is $B_y$,

an absolute value of a difference between a representative value $R_{ym}$ of a mean value of the amount y of the material and the lower limit tolerance is $A_{ym}$,

an absolute value of a difference between the representative value $R_{ym}$ and the upper limit tolerance is $B_{ym}$, and

the number of samples of the material is $N_y$,

values of $A_{ym}$, $B_{ym}$, and $R_{ym}$ are estimated with the following formulae.

[Math. 2]

$$A_{ym} = \frac{A_y}{\sqrt{N_y}}$$

$$B_{ym} = \frac{B_y}{\sqrt{N_y}}$$

$$R_{ym} = R_y$$

4. The estimation method according to claim 1, wherein provided that

an amount y of the material is influenced by other amounts $x_1$, $x_2$,..., $x_n$,

$$y = f(x_1, x_2, \ldots, x_n),$$

an absolute value of a difference between a representative value $R_{ym}$ of a mean value of the amount y and the lower limit tolerance is $A_{ym}$,
an absolute value of a difference between the representative value $R_{ym}$ and the upper limit tolerance is $B_{ym}$,
an absolute value of a difference between a representative value $R_{xm1}$, $R_{xm2}$,..., $R_{xmn}$ of a mean value of each of the amounts $x_1$, $x_2$,..., $x_n$ and the lower limit tolerance is $A_{xm1}$, $A_{xm2}$,..., $A_{xmn}$, and
an absolute value of a difference between the representative value $R_{xm1}$, $R_{xm2}$,..., $R_{xmn}$ and the upper limit tolerance is $B_{xm1}$, $B_{xm2}$,..., $B_{xmn}$,
values of $A_{ym}$, $B_{ym}$, and $R_{ym}$ are estimated with the following formulae.

[Math. 3]

$$A_{ym} = \sqrt{\sum_{i=1}^{n} \left( \frac{\partial y}{\partial x_i} \cdot A_{xmi} \right)^2}$$

$$B_{ym} = \sqrt{\sum_{i=1}^{n} \left( \frac{\partial y}{\partial x_i} \cdot B_{xmi} \right)^2}$$

$$R_{ym} = f(R_{xm1}, R_{xm2}, \cdots, R_{xmn})$$

5. The estimation method according to claim 1, wherein provided that

an amount y of the material is influenced by other amounts $x_1$, $x_2$,..., $x_n$,

$$y = f(x_1, x_2, \ldots, x_n),$$

an absolute value of a difference between a representative value $R_{ym}$ of a mean value of the amount y and the lower limit tolerance is $A_{ym}$,
an absolute value of a difference between the representative value $R_{ym}$ and the upper limit tolerance is $B_{ym}$,
an absolute value of a difference between a representative value $R_{xm1}$, $R_{xm2}$,..., $R_{xmn}$ of a mean value of each of the amounts $x_1$, $x_2$,..., $x_n$ and the lower limit tolerance is $A_{xm1}$, $A_{xm2}$, ... , $A_{xmn}$, and
an absolute value of a difference between the representative value $R_{xm1}$, $R_{xm2}$,..., $R_{xmn}$ and the upper limit tolerance is $B_{xm1}$, $B_{xm2}$,..., $B_{xmn}$,
values of $A_{ym}$, $B_{ym}$, and $R_{ym}$ are estimated with the following formulae.

[Math. 4]

$$A_{ym} = R_{ym}\sqrt{\sum_{i=1}^{n}\left(\frac{\partial y}{\partial x_i}\cdot\frac{A_{xmi}}{R_{xmi}}\right)^2}$$

$$B_{ym} = R_{ym}\sqrt{\sum_{i=1}^{n}\left(\frac{\partial y}{\partial x_i}\cdot\frac{B_{xmi}}{R_{xmi}}\right)^2}$$

$$R_{ym} = f(R_{xm1}, R_{xm2}, \cdots, R_{xmn})$$

6. The estimation method according to claim 5, wherein for a plurality of types of material samples having different mean values of the amounts x, provided that

an absolute value of a difference between a representative value $R_{Z1}$, $R_{Z2}$,..., $R_{Zn}$ of each of the mean values and the lower limit tolerance is $A_{Z1}$, $A_{Z2}$,..., $A_{Zn}$,
an absolute value of a difference between the representative value $R_{Z1}$, $R_{Z2}$,.. $R_{Zn}$ and the upper limit tolerance is $B_{Z1}$, $B_{Z2}$,..., $B_{Zn}$, and
the number of the material samples is $N_{Z1}$, $N_{Z2}$,..., $N_{Zn}$,
values of $A_{xm}/R_{xm}$ and $B_{xm}/R_{xm}$ are estimated with the following formulae.

[Math. 5]

$$\frac{A_{xm}}{R_{xm}} = \sqrt{\frac{\sum_{i=1}^{n}(N_{Zi}-1)\cdot\left(\frac{A_{Zi}}{R_{Zi}}\right)^2}{\sum_{i=1}^{n}(N_{Zi}-1)}}$$

$$\frac{B_{xm}}{R_{xm}} = \sqrt{\frac{\sum_{i=1}^{n}(N_{Zi}-1)\cdot\left(\frac{B_{Zi}}{R_{Zi}}\right)^2}{\sum_{i=1}^{n}(N_{Zi}-1)}}$$

**Patentansprüche**

1. Computer-implementiertes Schätzverfahren zum Schätzen einer Menge eines quantifizierten Materials, das durch eine Flüssigkeitströpfchenausstoßvorrichtung in einen Behälter eingespritzt wird, wobei die Menge des Materials eine Variation gemäß einer Wahrscheinlichkeitsverteilung aufweist und eine Wahrscheinlichkeit, dass die Menge des Materials innerhalb eines Bereichs einer unteren Grenztoleranz bis zu einer oberen Grenztoleranz auf der Wahrscheinlichkeitsverteilung liegt, größer als oder gleich einer Zielwahrscheinlichkeit ist, wobei das Schätzverfahren umfasst:

Berechnen der Wahrscheinlichkeitsverteilung, wobei das Berechnen der Wahrscheinlichkeitsverteilung das Berechnen der Wahrscheinlichkeitsverteilung unter Verwendung einer Leistungseigenschaft der Flüssigkeitströpfchenausstoßvorrichtung enthält, die dafür konfiguriert ist, das Material in den Behälter zu injizieren, einen ersten Schritt des Festlegens der Zielwahrscheinlichkeit;

einen zweiten Schritt des Berechnens des repräsentativen Wertes der Materialmenge auf der Wahrscheinlichkeitsverteilung,

einen dritten Schritt des Festlegens eines Intervalls von einer unteren Grenze bis zu einer oberen Grenze, das den repräsentativen Wert der Wahrscheinlichkeitsverteilung enthält;

einen vierten Schritt des Festlegens einer variablen Breite des Intervalls;

einen fünften Schritt des Vergrößerns einer Größe des Intervalls um die variable Breite von dem repräsentativen Wert in Richtung der unteren Grenze und Berechnen einer ersten Wahrscheinlichkeit, dass die Menge des Materials innerhalb des Intervalls mit der vergrößerten Größe liegt;

einen sechsten Schritt des Vergrößerns der Größe des Intervalls um die variable Breite von dem repräsentativen Wert in Richtung der oberen Grenze und Berechnen einer zweiten Wahrscheinlichkeit, dass die Menge des Materials innerhalb des Intervalls mit der vergrößerten Größe liegt; und

einen siebten Schritt des Festlegens der Größe des im fünften Schritt festgelegten Intervalls, wenn die erste Wahrscheinlichkeit größer oder gleich der zweiten Wahrscheinlichkeit ist, oder des Festlegens der Größe des im sechsten Schritt festgelegten Intervalls, wenn die erste Wahrscheinlichkeit kleiner als die zweite Wahrscheinlichkeit ist,

wobei:

der vierte Schritt, der fünfte Schritt, der sechste Schritt und der siebte Schritt wiederholt werden, bis die Wahrscheinlichkeit, dass die Menge des Materials innerhalb des Intervalls liegt, größer oder gleich der Zielwahrscheinlichkeit wird,

die untere Grenztoleranz die untere Grenze des Intervalls zu einem Zeitpunkt ist, zu dem die Wahrscheinlichkeit, dass die Menge des Materials innerhalb des Intervalls liegt, größer oder gleich der Zielwahrscheinlichkeit wird, und

die obere Grenztoleranz die obere Grenze des Intervalls zu einem Zeitpunkt ist, zu dem die Wahrscheinlichkeit, dass die Menge des Materials innerhalb des Intervalls liegt, größer oder gleich der Zielwahrscheinlichkeit wird; und

wobei das Material aus Nukleinsäuremolekülen besteht.

2. Schätzverfahren nach Anspruch 1, wobei, vorausgesetzt dass

eine Menge $y$ des Materials von anderen Mengen $x_1$, $x_2$,... $x_n$ beeinflusst wird,

$$y = f(x_1, x_2, \ldots, x_n),$$

ein absoluter Wert einer Differenz zwischen einem repräsentativen Wert $R_y$ der Menge $y$ und der unteren Grenztoleranz $A_y$ ist,

ein absoluter Wert einer Differenz zwischen der oberen Grenztoleranz und dem repräsentativen Wert $R_y$ $B_y$ ist,

ein absoluter Wert einer Differenz zwischen einem repräsentativen Wert $R_{xi}$ einer jeden der Menge $x_1$, $x_2$,... , $x_n$ und der unteren Grenztoleranz $A_{x1}$, $A_{x2}$,..., $A_{xn}$ ist, und

ein absoluter Wert einer Differenz zwischen der oberen Grenztoleranz und dem repräsentativen Wert $R_{xi}$ $B_{x1}$, $B_{x2}$,... , $B_{xn}$ ist,

die Werte von $A_y$, $B_y$ und $R_y$ mit den folgenden Formeln geschätzt werden.

[Math. 1]

$$A_y = \sqrt{\sum_{i=1}^{n}\left(\frac{\partial y}{\partial x_i} \cdot A_{xi}\right)^2}$$

$$B_y = \sqrt{\sum_{i=1}^{n}\left(\frac{\partial y}{\partial x_i} \cdot B_{xi}\right)^2}$$

$$R_y = f(R_{x1}, R_{x2}, \cdots, R_{xn})$$

3. Schätzverfahren nach Anspruch 1, wobei vorausgesetzt wird, dass

ein absoluter Wert einer Differenz zwischen einem repräsentativen Wert $R_y$ einer Menge y des Materials und der unteren Grenztoleranz $A_y$ ist,
ein absoluter Wert einer Differenz zwischen der oberen Grenztoleranz und dem repräsentativen Wert $R_y$ $B_y$ ist,
ein absoluter Wert einer Differenz zwischen einem repräsentativen Wert $R_{ym}$ eines Mittelwerts der Menge y des Materials und der unteren Grenztoleranz $A_{ym}$ ist,
ein absoluter Wert einer Differenz zwischen dem repräsentativen Wert $R_{ym}$ und der oberen Grenztoleranz $B_{ym}$ ist, und
die Anzahl der Proben des Materials $N_y$ ist,
die Werte von $A_{ym}$, $B_{ym}$ und $R_{ym}$ mit den folgenden Formeln geschätzt werden.

[Math. 2]

$$A_{ym} = \frac{A_y}{\sqrt{N_y}}$$

$$B_{ym} = \frac{B_y}{\sqrt{N_y}}$$

$$R_{ym} = R_y$$

4. Schätzverfahren nach Anspruch 1, wobei vorausgesetzt wird, dass

eine Menge y des Materials von anderen Mengen $x_1$, $x_2$,... , $x_n$, $y = f(x_1, x_2,... , x_n)$ beeinflusst wird,
ein absoluter Wert einer Differenz zwischen einem repräsentativen Wert $R_{ym}$ eines Mittelwerts der Menge y und der unteren Grenztoleranz $A_{ym}$ ist,
ein absoluter Wert einer Differenz zwischen dem repräsentativen Wert $R_{ym}$ und der oberen Grenztoleranz $B_{ym}$ ist,
ein absoluter Wert einer Differenz zwischen einem repräsentativen Wert $R_{xm1}$, $R_{xm2}$, ... , $R_{xmn}$ eines Mittelwerts jeder der Mengen $x_1$, $x_2$,... , $x_n$ und der unteren Grenztoleranz $A_{xm1}$, $A_{xm2}$,... , $A_{xmn}$ ist, und
ein absoluter Wert einer Differenz zwischen dem repräsentativen Wert $R_{xm1}$, $R_{xm2}$,... , $R_{xmn}$ und der oberen Grenztoleranz $B_{xm1}$, $B_{xm2}$, ... , $B_{xmn}$ ist,
die Werte von $A_{ym}$, $B_{ym}$ und $R_{ym}$ mit den folgenden Formeln geschätzt werden.

[Math. 3]

$$A_{ym} = \sqrt{\sum_{i=1}^{n} \left( \frac{\partial y}{\partial x_i} \cdot A_{xmi} \right)^2}$$

$$B_{ym} = \sqrt{\sum_{i=1}^{n} \left( \frac{\partial y}{\partial x_i} \cdot B_{xmi} \right)^2}$$

$$R_{ym} = f(R_{xm1}, R_{xm2}, \cdots, R_{xmn})$$

5. Schätzverfahren nach Anspruch 1, wobei vorausgesetzt wird, dass

eine Menge y des Materials von anderen Mengen $x_1$, $x_2$,... $x_n$ beeinflusst wird,

$$y = f(x_1, x_2, \ldots, x_n),$$

ein absoluter Wert einer Differenz zwischen einem repräsentativen Wert $R_{ym}$ eines Mittelwerts der Menge y und der unteren Grenztoleranz $A_{ym}$ ist,
ein absoluter Wert einer Differenz zwischen dem repräsentativen Wert $R_{ym}$ und der oberen Grenztoleranz $B_{ym}$ ist,
ein absoluter Wert einer Differenz zwischen einem repräsentativen Wert $R_{xm1}$, $R_{xm2}$, ... , $R_{xmn}$ eines Mittelwerts jeder der Mengen $x_1$, $x_2$,... , $x_n$ und der unteren Grenztoleranz $A_{xm1}$, $A_{xm2}$,... , $A_{xmn}$ ist, und
ein absoluter Wert einer Differenz zwischen dem repräsentativen Wert $R_{xm1}$, $R_{xm2}$,... , $R_{xmn}$ und der oberen Grenztoleranz $B_{xm1}$, $B_{xm2}$, ... , $B_{xmn}$ ist,
die Werte von $A_{ym}$, $B_{ym}$ und $R_{ym}$ mit den folgenden Formeln geschätzt werden.

[Math. 4]

$$A_{ym} = R_{ym} \sqrt{\sum_{i=1}^{n} \left( \frac{\partial y}{\partial x_i} \cdot \frac{A_{xmi}}{R_{xmi}} \right)^2}$$

$$B_{ym} = R_{ym} \sqrt{\sum_{i=1}^{n} \left( \frac{\partial y}{\partial x_i} \cdot \frac{B_{xmi}}{R_{xmi}} \right)^2}$$

$$R_{ym} = f(R_{xm1}, R_{xm2}, \cdots, R_{xmn})$$

6. Schätzverfahren nach Anspruch 5, wobei für eine Vielzahl von Typen von Materialproben mit unterschiedlichen Mittelwerten der Mengen x, vorausgesetzt, dass

ein absoluter Wert einer Differenz zwischen einem repräsentativen Wert $R_{Z1}$, $R_{Z2}$,... , $R_{Zn}$ von jedem der Mittelwerte und der unteren Grenztoleranz $A_{Z1}$, $A_{Z2}$,... , $A_{Zn}$ ist,
ein absoluter Wert einer Differenz zwischen dem repräsentativen Wert $R_{Z1}$, $R_{Z2}$,... , $R_{Zn}$ und der oberen Grenztoleranz $B_{Z1}$, $B_{Z2}$,... , $B_{Zn}$ ist, und
die Anzahl der Materialproben $N_{Z1}$, $N_{Z2}$,... , $N_{Zn}$ ist,

die Werte von $A_{xm}/R_{xm}$ und $B_{xm}/R_{xm}$ mit den folgenden Formeln geschätzt werden.

[Math. 5]

$$\frac{A_{xm}}{R_{xm}} = \sqrt{\frac{\sum_{i=1}^{n}(N_{Zi}-1)\cdot\left(\frac{A_{Zi}}{R_{Zi}}\right)^2}{\sum_{i=1}^{n}(N_{Zi}-1)}}$$

$$\frac{B_{xm}}{R_{xm}} = \sqrt{\frac{\sum_{i=1}^{n}(N_{Zi}-1)\cdot\left(\frac{B_{Zi}}{R_{Zi}}\right)^2}{\sum_{i=1}^{n}(N_{Zi}-1)}}$$

**Revendications**

1. Procédé d'estimation mis en oeuvre par ordinateur pour estimer une quantité d'une matière quantifiée injectée dans un récipient par un appareil d'éjection de gouttelettes de liquide, la quantité de matière ayant une variation selon une distribution de probabilité, et une probabilité que la quantité de matière se situe dans une plage d'une tolérance limite inférieure à une tolérance limite supérieure sur la distribution de probabilité étant supérieure ou égale à une probabilité cible, le procédé d'estimation comprenant :

   le calcul de la distribution de probabilité, le calcul de la distribution de probabilité comprenant le calcul de la distribution de probabilité à l'aide d'une propriété de performance de l'appareil d'éjection de gouttelettes de liquide configuré pour injecter le matériau dans le récipient,
   une première étape consistant à définir la probabilité cible ;
   une deuxième étape consistant à calculer la valeur représentative de la quantité de matériau sur la distribution de probabilité ;
   une troisième étape consistant à définir un intervalle d'une limite inférieure à une limite supérieure comprenant la valeur représentative sur la distribution de probabilité ;
   une quatrième étape consistant à définir une largeur variable de l'intervalle ;
   une cinquième étape consistant à augmenter une taille de l'intervalle de la largeur variable depuis la valeur représentative vers la limite inférieure, et à calculer une première probabilité que la quantité de matériau se trouve dans l'intervalle avec la taille augmentée ;
   une sixième étape consistant à augmenter la taille de l'intervalle de la largeur variable depuis la valeur représentative vers la limite supérieure, et à calculer une deuxième probabilité que la quantité de matériau se trouve dans l'intervalle avec la taille augmentée ; et
   une septième étape consistant à fixer la taille de l'intervalle défini à la cinquième étape si la première probabilité est supérieure ou égale à la deuxième probabilité, ou à fixer la taille de l'intervalle défini à la sixième étape si la première probabilité est inférieure à la deuxième probabilité,
   dans lequel :

      la quatrième étape, la cinquième étape, la sixième étape et la septième étape sont répétées jusqu'à ce que la probabilité que la quantité de matériau se trouve dans l'intervalle devienne supérieure ou égale à la probabilité cible,
      la tolérance limite inférieure est la limite inférieure de l'intervalle à un instant où la probabilité que la quantité de matériau se trouve dans l'intervalle devient supérieure ou égale à la probabilité cible, et
      la tolérance limite supérieure est la limite supérieure de l'intervalle à un instant où la probabilité que la quantité de matière se trouve dans l'intervalle devient supérieure ou égale à la probabilité cible ; et
      le matériau étant constitué de molécules d'acide nucléique.

2. Procédé d'estimation selon la revendication 1, dans lequel à condition

   qu'une quantité y du matériau est influencée par d'autres quantités $x_1$, $x_2$,..., $x_n$,

$$y = f(x_1, x_2,..., x_n),$$

qu'une valeur absolue d'une différence entre une valeur représentative $R_y$ de la quantité y et la tolérance limite inférieure est $A_y$,

qu'une valeur absolue d'une différence entre la tolérance limite supérieure et la valeur représentative $R_y$ est $B_y$,

qu'une valeur absolue d'une différence entre une valeur représentative $R_{xi}$ de chacune des quantités $x_1$, $x_2$,..., $x_n$ et la tolérance limite inférieure est $A_{xi}$, $A_{x2}$,..., $A_{xn}$, et

qu'une valeur absolue d'une différence entre la tolérance limite supérieure et la valeur représentative $R_{xi}$ est $B_{x1}$, $B_{x2}$,..., $B_{xn}$,

les valeurs de $A_y$, $B_y$ et $R_y$ sont estimées avec les formules suivantes.

[Math. 1]

$$A_y = \sqrt{\sum_{i=1}^{n}\left(\frac{\partial y}{\partial x_i} \cdot A_{xi}\right)^2}$$

$$B_y = \sqrt{\sum_{i=1}^{n}\left(\frac{\partial y}{\partial x_i} \cdot B_{xi}\right)^2}$$

$$R_y = f(R_{x1}, R_{x2}, \cdots, R_{xn})$$

3. Procédé d'estimation selon la revendication 1, dans lequel à condition

qu'une valeur absolue d'une différence entre une valeur représentative $R_y$ d'une quantité y du matériau et la tolérance limite inférieure est $A_y$,

qu'une valeur absolue d'une différence entre la tolérance limite supérieure et la valeur représentative $R_y$ est $B_y$,

qu'une valeur absolue d'un écart entre une valeur représentative $R_{ym}$ d'une valeur moyenne de la quantité y du matériau et la tolérance limite inférieure est $A_{ym}$,

qu'une valeur absolue d'une différence entre la valeur représentative $R_{ym}$ et la tolérance limite supérieure est $B_{ym}$, et

que le nombre d'échantillons du matériau est $N_y$,

les valeurs de $A_{ym}$, $B_{ym}$ et $R_{ym}$ sont estimées avec les formules suivantes.

[Math. 2]

$$A_{ym} = \frac{A_y}{\sqrt{N_y}}$$

$$B_{ym} = \frac{B_y}{\sqrt{N_y}}$$

$$R_{ym} = R_y$$

4. Procédé d'estimation selon la revendication 1, dans lequel à condition

qu'une quantité y du matériau est influencée par d'autres quantités $x_1$, $x_2$,..., $x_n$,

$$y = f(x_1, x_2,..., x_n),$$

qu'une valeur absolue d'un écart entre une valeur représentative $R_{ym}$ d'une valeur moyenne de la quantité y et la tolérance limite inférieure est $A_{ym}$,
qu'une valeur absolue d'une différence entre la valeur représentative $R_{ym}$ et la tolérance limite supérieure est $B_{ym}$,
qu'une valeur absolue d'une différence entre une valeur représentative $R_{xm1}$, $R_{xm2}$,..., $R_{xmn}$ d'une valeur moyenne de chacune des quantités $x_1$, $x_2$,..., $x_n$ et la tolérance limite inférieure est $A_{xm1}$, $A_{xm2}$,... , $A_{xmn}$ et
qu'une valeur absolue d'une différence entre la valeur représentative $R_{xm1}$, $R_{xm2}$,..., $R_{xmn}$ et la tolérance limite supérieure est $B_{xm1}$, $B_{xm2}$,..., $B_{xmn}$,
les valeurs de $A_{ym}$, $B_{ym}$ et $R_{ym}$ sont estimées avec les formules suivantes.

[Math. 3]

$$A_{ym} = \sqrt{\sum_{i=1}^{n}\left(\frac{\partial y}{\partial x_i}\cdot A_{xmi}\right)^2}$$

$$B_{ym} = \sqrt{\sum_{i=1}^{n}\left(\frac{\partial y}{\partial x_i}\cdot B_{xmi}\right)^2}$$

$$R_{ym} = f(R_{xm1}, R_{xm2}, \cdots, R_{xmn})$$

5. Procédé d'estimation selon la revendication 1, dans lequel à condition

qu'une quantité y du matériau est influencée par d'autres quantités $x_1$, $x_2$,..., $x_n$,

$$y = f(x_1, x_2,..., x_n),$$

qu'une valeur absolue d'un écart entre une valeur représentative $R_{ym}$ d'une valeur moyenne de la quantité y et la tolérance limite inférieure est $A_{ym}$,
qu'une valeur absolue d'une différence entre la valeur représentative $R_{ym}$ et la tolérance limite supérieure est $B_{ym}$,
qu'une valeur absolue d'une différence entre une valeur représentative $R_{xm1}$, $R_{xm2}$,..., $R_{xmn}$ d'une valeur moyenne de chacune des quantités $x_1$, $x_2$,..., $x_n$ et la tolérance limite inférieure est $A_{xm1}$, $A_{xm2}$,... , $A_{xmn}$ et
qu'une valeur absolue d'une différence entre la valeur représentative $R_{xm1}$, $R_{xm2}$,..., $R_{xmn}$ et la tolérance limite supérieure est $B_{xm1}$, $B_{xm2}$,..., $B_{xmn}$,
les valeurs de $A_{ym}$, $B_{ym}$ et $R_{ym}$ sont estimées avec les formules suivantes.

[Math. 4]

$$A_{ym} = R_{ym} \sqrt{\sum_{i=1}^{n} \left( \frac{\partial y}{\partial x_i} \cdot \frac{A_{xmi}}{R_{xmi}} \right)^2}$$

$$B_{ym} = R_{ym} \sqrt{\sum_{i=1}^{n} \left( \frac{\partial y}{\partial x_i} \cdot \frac{B_{xmi}}{R_{xmi}} \right)^2}$$

$$R_{ym} = f(R_{xm1}, R_{xm2}, \cdots, R_{xmn})$$

6. Procédé d'estimation selon la revendication 5, dans lequel pour une pluralité de types d'échantillons de matériaux ayant des valeurs moyennes différentes des quantités x, à condition

qu'une valeur absolue d'un écart entre une valeur représentative $R_{z1}$, $R_{z2}$,..., $R_{zn}$ de chacune des valeurs moyennes et la tolérance limite inférieure est $A_{z1}$, $A_{z2}$,..., $A_{zn}$,
qu'une valeur absolue d'une différence entre la valeur représentative $R_{z1}$, $R_{z2}$,..., $R_{zn}$ et la tolérance limite supérieure est $B_{z1}$, $B_{z2}$,..., $B_{zn}$, et
le nombre d'échantillons de matériau est $N_{z1}$, $N_{z2}$,..., $N_{zn}$,
les valeurs de $A_{xm}/R_{xm}$ et $B_{xm}/R_{xm}$ sont estimées avec les formules suivantes.

[Math. 5]

$$\frac{A_{xm}}{R_{xm}} = \sqrt{\frac{\sum_{i=1}^{n}(N_{zi}-1) \cdot \left(\frac{A_{zi}}{R_{zi}}\right)^2}{\sum_{i=1}^{n}(N_{zi}-1)}}$$

$$\frac{B_{xm}}{R_{xm}} = \sqrt{\frac{\sum_{i=1}^{n}(N_{zi}-1) \cdot \left(\frac{B_{zi}}{R_{zi}}\right)^2}{\sum_{i=1}^{n}(N_{zi}-1)}}$$

# FIG. 1

# FIG. 2A

# FIG. 2B

± 2σ ON
NORMAL DISTRIBUTION

# FIG. 3

START

Set target probability α — S301

Compute representative value — S302

Set initial interval [representative value - a, representative value + b] — S303

Set variable width w — S304

Compute probability (first probability) of concentration being within interval [representative value - (a + w), representative value + b] — S305

Compute probability (second probability) of concentration being within interval [representative value - a, representative value + (b + w)] — S306

First probability ≥ Second probability? — S307-1

S307-2 / Y

Fix [representative value - (a + w), representative value + b] — S307-2

N / S307-3

Fix [representative value - a, representative value + (b+w)] — S307-3

Estimated probability ≥ Target probability? — S308

N

Y

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018096636 A
- WO 2006030822 A
- JP 2004257809 A